(19) Europäisches Patentamt
European Patent Office
Office européen des brevets

(11) Veröffentlichungsnummer: **0 236 884 B1**

# (12) EUROPÄISCHE PATENTSCHRIFT

(45) Veröffentlichungstag der Patentschrift: **27.12.90**

(21) Anmeldenummer: **87102872.6**

(22) Anmeldetag: **28.02.87**

(51) Int. Cl.[5]: **C07D 249/08,** C07D 301/14, C07D 303/18, C07C 43/215, C07C 43/285

## (54) Verfahren zur Herstellung von Phenoxyalkanoltriazolverbindungen und Zwischenprodukte hierfür.

(30) Priorität: **04.03.86 DE 3606947**

(43) Veröffentlichungstag der Anmeldung: **16.09.87 Patentblatt 87/38**

(45) Bekanntmachung des Hinweises auf die Patenterteilung: **27.12.90 Patentblatt 90/52**

(84) Benannte Vertragsstaaten: **AT BE CH DE ES FR GB GR IT LI NL SE**

(56) Entgegenhaltungen:
**EP-A- 0 122 056**
**DE-A- 3 321 023**
**DE-A- 3 416 444**
**DE-A- 3 420 227**

**CHEMICAL ABSTRACTS, Band 105, Nr. 1, 7. Juli 1986, Columbus, Ohio, USA J. GASTEIGER, KH. KAUFMANN, Chr. HERZIG, T.W. BENTLEY: "Nucleophilic substitution at a saturated carbon atom with retention of configuration: the reaction of trans-2-halo-3-tert-butyloxiranes with phenolates" Seite 595, Spalte 1, Zusammenfassung Nr. 6362c 000**
**CHEMICAL ABSTRACTS, Band 95, Nr. 24, 14. Dezember, 1981, Columbus, Ohio, USA J. MULZER, M. ZIPPEL: "Acid catalysis induces a total change from retention to inversion of configuration in carbon dioxide**

(73) Patentinhaber: **BASF Aktiengesellschaft, Carl-Bosch-Strasse 38, D-6700 Ludwigshafen(DE)**

(72) Erfinder: **Schulz, Guenter, Dr., Carl-Boch-Strasse 96, D-6700 Ludwigshafen(DE)**
Erfinder: **Trautmann, Walter, Dr., Ritterbueschel 87, D-6730 Neustadt(DE)**
Erfinder: **Sauter, Hubert, Dr., Neckarpromenade 20, D-6800 Mannheim 1(DE)**
Erfinder: **Mackenroth, Wolfgang, Dr., Seebacher Strasse 37, D-6702 Bad Duerkheim(DE)**

(56) Entgegenhaltungen: (Fortsetzung)
**elimination from beta-lactones" Seite 476, Spalte 2, Zusammenfassung Nr. 219687zeite 595, Spalte 1, Zusammenfassung Nr. 6362c 000**
**CHEMICAL ABSTRACTS, Band 94, Nr. 8, 23. Februar 1981, Columbus, Ohio, USA W.S. MURPHY, S. WILLIAM: "Intramolecular alkylation of phenols. Part 4. Base-catalyzed cyclization of phenolic enones. Scope and limitations" Seite 686, Spalte 2, Zusammenfassung Nr. 65247wassung Nr. 219687zeite 595, Spalte 1, Zusammenfassung Nr. 6362c 000**

EP 0 236 884 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Phenoxyalkanol-triazolverbindungen in Form ihrer weitgehend reinen Diastereomeren, Zwischenprodukte hierfür und Verfahren zur Herstellung der Zwischenprodukte in Form ihrer weitgehend reinen Isomeren.

Es is bekannt, Phenoxyalkanol-triazolverbindungen z.B. das 8-(2-Fluorphenoxy)-4-(1,2,4-triazol-1-yl)-3-hydroxy-2,2-dimethyl-octan als Fungizide zu verwenden (EP 40 350). Bedingt durch zwei chirale Zentren im Molekül treten diese Verbindungen in Form von optischen Isomeren und als Diastereomere auf. Die fungizide Wirkung der diastereomeren Enantiomerenpaare ist im allgemeinen nicht gleich, so daß ein Interesse daran besteht, das wirksamere Diastereomere herzustellen. Es ist bekannt, dies durch selektive Reduktion der entsprechenden Triazolylketone herzustellen (DE 33 21 023.3). Die dabei erzielten chemischen Ausbeuten sowie die erreichte Isomeren-Reinheit der Diastereomeren ist jedoch nicht immer befriedigend.

Es wurde nun gefunden, daß man eine Verbindung der Formel I

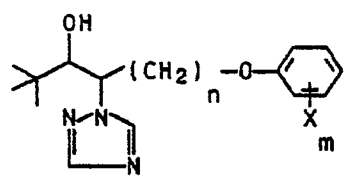

I

in der
X für Wasserstoff, Fluor, Chlor, Brom, $C_1$-$C_3$-Alkyl, $C_1$-$C_3$-Alkoxy, Trifluormethyl, Phenyl oder Phenoxy steht,
m eine ganze Zahl von 1 bis 3 ist, wobei die einzelnen Atome oder Gruppen gleich oder verschieden sind, wenn m größer als 1 ist, und n eine ganze Zahl von 2 bis 6 bedeutet,
in Form ihrer weitgehend reinen Diastereomeren erhält, wenn man ein Alkenoxid der Formel II

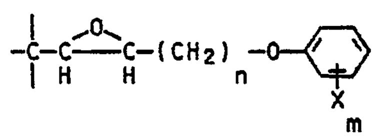

II

in der X und m die oben genannten Bedeutungen haben, in Form seines weitgehend reinen E- oder Z-Isomeren mit Triazol in Gegenwart eines Säureamids und einer alkalischen Verbindung bei einer Temperatur von 130 bis 230°C umsetzt.

Ein weitgehend reines Diastereomer enthält 95 % (Gew.%) oder mehr des Diastereomers, bezogen auf den Gesamtgehalt an Diastereomeren.

Ein $C_1$-$C_3$-Alkyl ist beispielsweise Methyl, Ethyl, Propyl, Isopropyl. Ein $C_1$-$C_3$-Alkoxy ist beispielsweise Methoxy, Ethoxy, Propoxy, Isopropoxy. Man arbeitet bei der Umsetzung in Gegenwart von Alkali- oder Erdalkalihydroxiden oder vergleichbaren alkalischen Verbindungen, z.B. Natriumhydroxid, Kaliumhydroxid, Natriumcarbonat, Kaliumcarbonat, in Säureamiden, insbesondere in N-Methylpyrrolidon oder Dimethylformamid als Verdünnungsmittel und setzt bei Temperaturen zwischen 130 und 230°C um. Bei dieser Umsetzung bleibt überraschend die räumliche Orientierung der verschiedenen Substituenten an den chiralen Zentren unverändert erhalten. Die räumliche Struktur des Endprodukts ist also die gleiche wie die des für die Umsetzung verwendeten Alkenoxids. Auf diese Weise gelingt es, durch eine einfache Umsetzung Endprodukte definierter räumlicher Struktur zu erhalten. Bei guter Ausbeute bezogen auf die chemische Umsetzung erhält man glatt ein im Hinblick auf den Isomerengehalt mindestens 95 %iges Diastereomer.

Das für die Umsetzung benötigte Alkenoxid erhält man, indem man ein Alken der Formel III

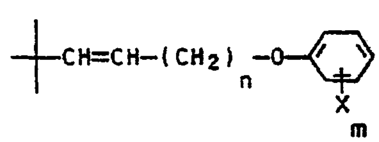

III

in der X und m die oben genannten Bedeutungen haben, in Form seines weitgehend reinen E- oder Z-Isomeren mit einer organischen Persäure in Gegenwart einer organischen Flüssigkeit bei einer Temperatur von -10 bis +30°C, oxydiert. Eine organische Persäure ist beispielsweise m-Chlorperbenzoesäure oder Peressigsäure. Dabei arbeitet man beispielsweise in chlorierten Kohlenwasserstoffen - insbesondere in

Methylenchlorid, wenn mit m-Chlorperbenzoesäure oxydiert wird - oder in protischen Lösungsmitteln - insbesondere in wäßriger Essigsäure, wenn mit Peressigsäure oxydiert wird. Im Falle der Oxydation mit Peressigsäure kann diese in situ aus $H_2O_2$ und Essigsäure hergestellt werden. Die Oxydation wird bei Temperaturen von -10 bis 30°C durchgeführt, insbesondere im Temperaturbereich von +15 bis +25°C.

Die beiden Verfahrensschritte Oxydation und Umsetzung mit Triazol verlaufen bei sehr guter chemischer Ausbeute mit Selektivitäten bezogen auf die Isomeren von jeweils mindestens 95 %. Auf diese Weise erhält man glatt aus den E-Alkenen der Formel III das RS/SR Enantiomerenpaar der Formel I und aus den Z-Alkenen der Formel III erhält man das RR/SS-Enantiomerenpaar der Formel I.

Das Problem der diastereoselektiven Synthese von I reduziert sich damit auf die Synthese von stereochemisch einheitlichen E- oder Z-Alkenen der Formel III.

Die Alkene der Formel III

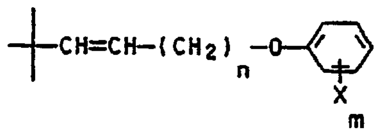

III

in der
X für Wasserstoff, Fluor, Chlor, Brom, $C_1$-$C_3$-Alkyl, $C_1$-$C_3$-Alkoxy, Trifluormethyl, Phenyl oder Phenoxy steht,
m eine ganze Zahl von 1 bis 3 ist, wobei die einzelnen Atome oder Gruppen gleich oder verschieden sind, wenn m größer als 1 ist, und n eine ganze Zahl von 2 bis 6 bedeutet,
in Form ihrer weitgehend reinen E-Isomeren erhält man, wenn man eine Verbindung der Formel IV

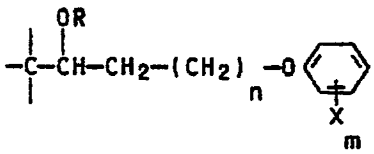

IV

in der
X für Wasserstoff, Fluor, Chlor, Brom, $C_1$-$C_3$-Alkyl, $C_1$-$C_3$-Alkoxy, Trifluormethyl, Phenyl oder Phenoxy steht,
m eine ganze Zahl von 1 bis 3 ist, wobei die einzelnen Atome oder Gruppen gleich oder verschieden sind, wenn m größer als 1 ist, und n eine ganze Zahl von 2 bis 6 bedeutet, und
R den Rest $-COCH_3$ oder $-CSSCH_3$ bedeutet
auf eine Temperatur von 200 bis 400°C oder 100 bis 300°C erhitzt.

Im Falle der Acetate wird die Substanz als solche oder in einem hochsiedenden inerten Verdünnungsmittel auf Temperaturen von 200 bis 400°C erhitzt. Für das Durchführen der Reaktion in Substanz seien insbesondere Temperaturen von 250 bis 350°C genannt. Im Falle der Xanthogenate genügt es, die Verbindung auf Temperaturen von 100 bis 300°C zu erhitzen. Man arbeitet dabei in einem hochsiedenden Verdünnungsmittel oder erhitzt die Substanz als solche. Für das Durchführen der Reaktion in Substanz seien Temperaturen von 150 bis 250°C genannt. Die entstehenden Alkene der Formel III werden unter Normaldruck oder vermindertem Druck destilliert. Die chemischen Ausbeuten liegen zwischen 90 und 100 %; die Selektivität bezogen auf Isomere liegt mindestens bei 95 %. Die Herstellung der Acetate oder Xanthogenate kann auf üblichem Weg aus den Alkoholen, die in DE 34 00 829 beschrieben sind, erfolgen.

Ein anderer Weg zur Herstellung der Alkene der Formel III in Form ihrer weitgehend reinen E-Isomeren besteht darin, daß man Alkene der Formel III, die in Form ihrer Z-Isomeren vorliegen, mit kurzwelligem Licht in Gegenwart von Diphenyldisulfid und einem Kohlenwasserstoff behandelt. Ein Kohlenwasserstoff ist beispielsweise ein Alkan oder Cycloalkan mit 5 bis 8 Kohlenstoffatomen, z.B. Pentan, Hexan, Cyclohexan, Heptan oder Oktan. Kurzwelliges Licht ist beispielsweise Licht, das die spektrale Verteilung einer Quecksilberdampfhochdrucklampe hat. Die Behandlung kann bei üblichen Temperaturen, z.B. Raumtemperatur (20°C) oder bei einer beliebigen anderen Temperatur erfolgen.

Die Alkene der Formel III in Form ihrer weitgehend reinen Z-Isomeren erhält man, wenn man Pivaladehyd der Formel $(CH_3)_3C-CHO$ mit einer Verbindung der Formel V

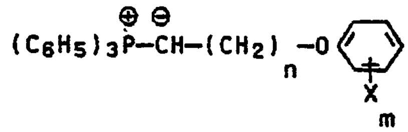 V

in der
X für Wasserstoff, Fluor, Chlor, Brom, $C_1$-$C_3$-Alkyl, $C_1$-$C_3$-Alkoxy, Trifluormethyl, Phenyl oder Phenoxy steht,
m eine ganze Zahl von 1 bis 3 ist, wobei die einzelnen Atome oder Gruppen gleich oder verschieden sind, wenn m größer als 1 ist, und n eine ganze Zahl von 2 bis 6 bedeutet,
umsetzt. Die Umsetzung erfolgt unter salzfreien Bedingungen, d.h. in polar aprotischen Lösungsmitteln unter Abwesenheit von gelösten Metallkationen (vgl. H.O. House, Modern Synthetic Reactions, 2nd ed. 1972, S. 707f.).

Als Lösungsmittel sind dabei lineare oder cyclische Ether geeignet, insbesondere wird Tetrahydrofuran genannt. Die Umsetzung läuft bei Temperaturen von 20 bis 100°C ab, für den Fall, daß Tetrahydrofuran als Lösungsmittel verwendet wird, werden insbesondere Temperaturen von 50 bis 60°C (Rückflußtemperatur) genannt. Nach Beendigung der Umsetzung und dem Abtrennen von Triphenylphosphinoxid erhält man das Z-Alken der Formel III in guten chemischen Ausbeuten bei einer Selektivität von mindestens 90 % an Z-Isomeren. Die zur Herstellung der Phosphorverbindung benötigten Vorprodukte sind bekannt (EP 40 350).

Beispiele

Am Beispiel der Herstellung von 8-(2-Fluorphenoxy)-4-(1,2,4-triazol-1-yl)-3-hydroxy-2,2-dimethyloctan 3R,4S/3S,4R-Enantiomerenpaar werden die einzelnen Verfahrensschritte exemplarisch dargestellt. Das in den Beispielen beschriebene Verfahren kann verwendet werden, um die in den Tabellen aufgeführten Verbindungen zu synthetisieren.

Herstellung des Acetats

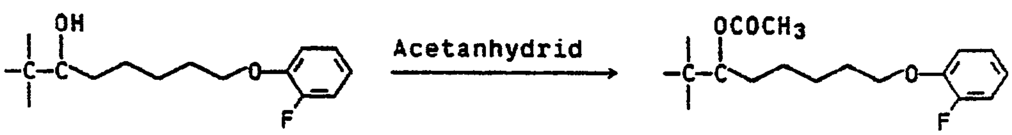


58 g (0.23 Mol) des Octanol-Derivats werden in 200 ml Essigsäureanhydrid unter Zusatz von 1 ml konz. Salzsäure 2 h zum Rückfluß erhitzt. Destillation liefert das Acetat nach einem Essigsäure/Essigsäureanhydrid Vorlauf $Kp_{0.05}$: 135°C (65 g)
$^1$H-NMR ($CDCl_3$):
0,9 s 9H; 1.25-1.65 m 6 H; 1.8 m 2 H;
2.05 s 3H; 4.0 t 2 H; 4.75 dd 1H; 6.8 - 7.1 m 4H.

Tabelle 1

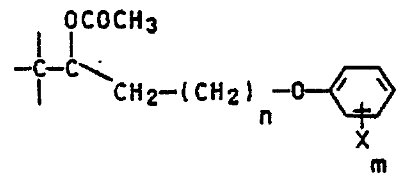

| Nr. | $X_m$ | n | Physikalische Daten |
|---|---|---|---|
| IV/1 | 2-F | 4 | Kp. (0.05) 135°C |
| IV/2 | 2-Cl | 4 | |
| IV/3 | $2\text{-}CH_3$ | 4 | |
| IV/4 | 4-F | 4 | |
| IV/5 | 4-Cl | 4 | |
| IV/6 | $4\text{-}CH_3$ | 4 | |
| IV/7 | $2,4\text{-}Cl_2$ | 4 | |
| IV/8 | $2,4,6\text{-}Cl_3$ | 4 | |

Herstellung des Xanthogenats

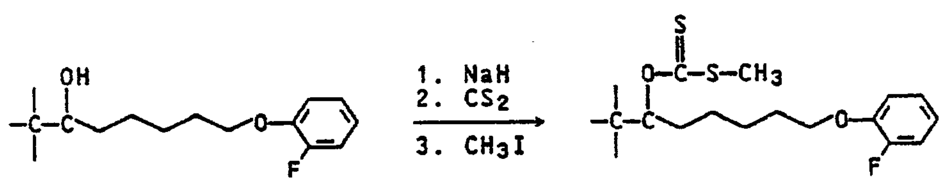


58 g (0.23 Mol) des Oktanol-Derivats gelöst in Toluol werden portionsweise mit insgesamt 1 Äquivalent Natriumhydrid versetzt. Zur Vervollständigung der Alkoholat-Bildung wird 3 h auf Rückflußtemperatur erwärmt. Man läßt auf 20°C abkühlen und tropft $CS_2$ gelöst in 100 ml Diethylether zu. Etwa 1 h nach beendeter $CS_2$-Zugabe tropft man etwa 1,5 Äquivalente Methyliodid zu und erhitzt 2 h zum Rückfluß. Nach Filtration und Abdampfen aller leichtflüchtigen Anteile im Vakuum verbleiben 77 g des Xanthogenats als nahezu farbloses Öl.

$^1$-NMR ($CDCl_3$):
0.95 s 9H; 1.3-1.9 m 8H; 2.55 s 3H; 4.0 t 2H,
5.72 dd 1H; 6.8-7.3 m 4H.
IR (Aufstrich):
2951, 1507, 1456, 1281, 1223, 1206, 1110, 1051 $cm^{-1}$.

Tabelle 2

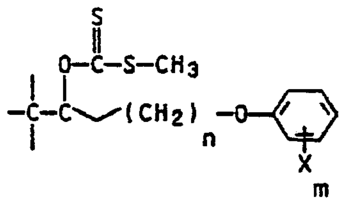

| Nr. | $X_m$ | n | Physikalische Daten |
|---|---|---|---|
| IV/ 9 | 2-F | 4 | NMR/JR siehe Beispiel |
| IV/10 | 2-Cl | 4 | |
| IV/11 | 2-$CH_3$ | 4 | |
| IV/12 | 4-F | 4 | |
| IV/13 | 4-Cl | 4 | |
| IV/14 | 4-$CH_3$ | 4 | |
| IV/15 | 2,4-$Cl_2$ | 4 | |
| IV/16 | 2,4,6-$Cl_3$ | 4 | |

Herstellung des Alkens aus dem Acetat

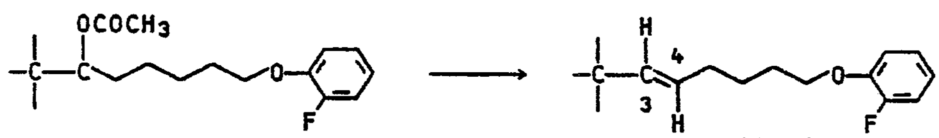

IIIa/1

81 g (0.26 Mol) des Acetats IV/1 werden im Metallbad auf 350°C erhitzt. Über eine aufgesetzte Kolonne destilliert zunächst Essigsäure und dann das Alkan IIIa/1 ab. Man erhält 64 g des Alkens $Kp_{760}$ 295°C. Die E-Konfiguration der Doppelbindung kann durch die Lage der Kohlenstoffe 3 und 4 in $^{13}C$-NMR (in $CDCl_3$) einwandfrei identifiziert werden:

C-3 142,1 ppm
C-4 124,2 ppm

Herstellung des Alkens aus dem Xanthogenat

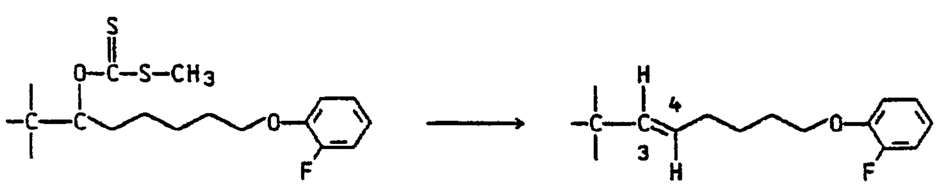

IIIa/1

20 g des Xanthogenats IV/9 (0.057 Mol) werden im leichten $N_2$-Strom auf ca. 200°C erhitzt. Der $N_2$-Strom treibt im Verlauf von 2 h 10.3 g des Alkens IIIa/1 in die Vorlage. Die Konfiguration der Doppelbindung kann nach $^{13}C$-NMR eindeutig als E ermittelt werden:

C-3 142,1 ppm
C-4 124,2 ppm

Herstellung des Alkens IIIa/1 durch Isomerisierung

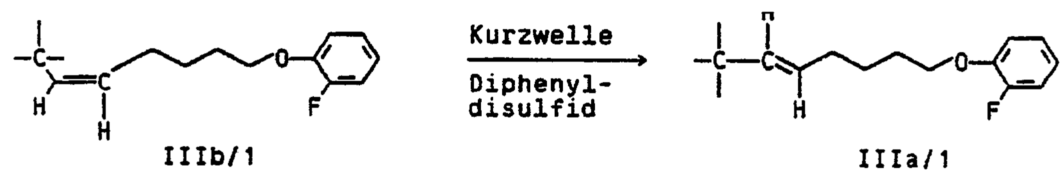


IIIb/1 IIIa/1

33 g (0,132 mol) Z-8-(2-Fluorphenoxy)-2,2-dimethyl-octen-3 IIIb/1 werden in 400 ml Cyclohexan gelöst. 2,83 g (0,013 mol) Diphenyldisulfid werden zugegeben und die Lösung 30 min mit Stickstoff gespült. Anschließend wird die Lösung mit einer Quecksilberhochdrucklampe (125 W-Philips HPK) durch ein Duranfilter bei 35°C Innentemperatur bestrahlt. Nach 3 h Belichtungszeit ist der Umsatz 95 %. Die Reaktionslösung wird filtriert und über eine Kolonne destilliert. Man erhält 25,8 g E-8-(2-Fluorphenoxy)-2,2-dimethyl-octen-3 IIIa/1 mit Siedepunkt 0,2 mbar/90°C.

Tabelle 3a

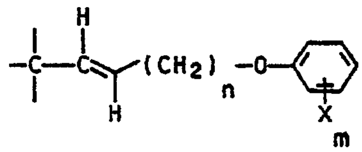

E-Konfiguration

| Nr. | $X_m$ | n | Physikalische Daten |
|---|---|---|---|
| IIIa/1 | 2-F | 4 | $Kp_{760}$ 295°C |
| IIIa/2 | 2-Cl | 4 | |
| IIIa/3 | 2-$CH_3$ | 4 | |
| IIIa/4 | 4-F | 4 | |
| IIIa/5 | 4-Cl | 4 | |
| IIIa/6 | 4-$CH_3$ | 4 | |
| IIIa/7 | 2,4-$Cl_2$ | 4 | |
| IIIa/8 | 2,4,6-$Cl_3$ | 4 | |

Herstellung des Alkens IIIb/1

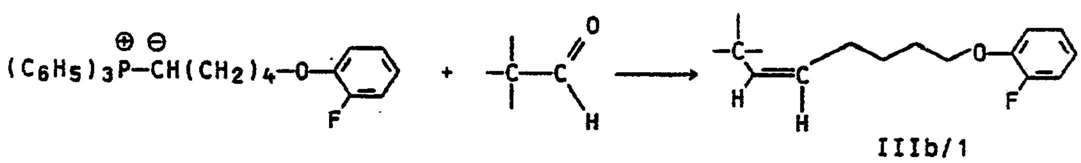

IIIb/1

261,5 g (0,5 mol) Triphenyl-[5-(3-fluorphenoxy)-pentyl-]-phosphoniumbromid werden aus äquimolaren Mengen Triphenylphosphin und 5-(2-Fluorphenoxy)-brompentan bei 130 bis 140°C hergestellt. Nach dem Erkalten wird die erstarrte Schmelze zerkleinert und in 1000 ml Tetrahydrofuran suspendiert. Unter Kühlen tropft man 370 ml (0,55 mol) Butyllithium-Lösung 1,5 molar in n-Hexan hinzu. Man hält die Innentemperatur bei 20 bis 30°C und rührt noch 1 h nach. Zu der entstandenen braunen Lösung tropft man nun in ca. 30 min. eine Lösung von 43 g (0,5 mol) Pivalaldehyd in 150 ml Tetrahydrofuran. Man rührt 20 h bei 20°C und kocht 24 h bei 60°C am Rückfluß bis ein weißer, körniger Niederschlag entsteht.

Die Mischung wird mit 2 l Wasser hydrolysiert, die organische Phase wird abgetrennt, die wäßrige Phase noch 3mal mit Diethylether extrahiert. Die vereinigten organischen Phasen werden 2mal mit Was-

ser gewaschen, über $Na_2SO_4$ getrocknet und eingedampft. Den Abdampfrückstand versetzt man mit 250 ml Ether und fällt Triphenylphosphinoxid aus. Man saugt ab und destilliert das Filtrat. Man erhält 33,5 g Z-8-(2-Fluorphenoxy)-2,2-dimethyl-octen(-3-) IIIb/1 als farblose Flüssigkeit mit Siedepunkt 2 mbar/127°C.

Tabelle 3b

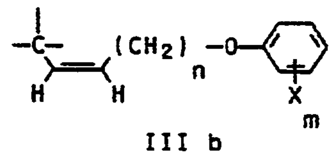

Z-Konfiguration

III b

| Nr. | $X_m$ | n | Physikalische Daten |
|---|---|---|---|
| IIIb/1 | 2-F | 4 | Kp (2 mbar): 127°C |
| IIIb/2 | 2-Cl | 4 | |
| IIIb/3 | $2-CH_3$ | 4 | |
| IIIb/4 | 4-F | 4 | |
| IIIb/5 | 4-Cl | 4 | |
| IIIb/6 | $4-CH_3$ | 4 | |
| IIIb/7 | $2,4-Cl_2$ | 4 | |
| IIIb/8 | $2,4,6-Cl_3$ | 4 | |

Herstellung des Alkenoxids

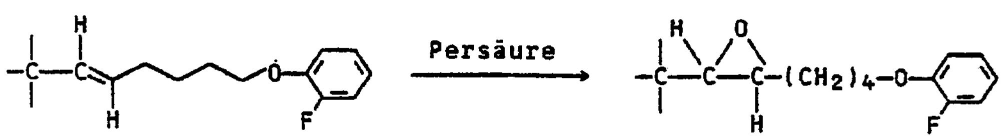


IIIa/1 IVa/1

50 g (0.2 mol) E-8-(2-Fluorphenoxy)-2,2-dimethyl-octen-3 IIIa/1 werden in 300 ml Methylenchlorid gelöst. Unter Rühren tropft man eine Lösung von 39,7 g (0,23 mol) 3-Chlorperbenzoesäure in 550 ml Methylenchlorid innerhalb 20 min. zu und hält die Temperatur durch Kühlen bei 20°C. Allmählich fällt weißer Niederschlag (3-Chlorbenzoesäure) aus. Man rührt 2 Tage bei 20°C. Danach ist das Ausgangsmaterial vollständig umgesetzt (gaschromatographische Analyse). Zur Aufarbeitung rührt man mit 1000 ml 10%iger wäßriger Natriumsulfit-Lösung bis kein Peroxid mehr nachweisbar ist. Mit Natronlauge stellt man pH = 7 ein, trennt die Methylenchloridphase ab und extrahiert die wäßrige Phase noch einmal mit Methylenchlorid. Die vereinigten organischen Phasen werden 2mal mit Natriumcarbonat-Lösung und 2mal mit dest. Wasser gewaschen, über Natriumsulfat getrocknet und am Rotationsverdampfer im Vakuum bei 40°C eingedampft. Man erhält 55,6 g Rohprodukt mit einem gaschromatographisch bestimmten Gehalt von 90 % 2-[4-(2-Fluorphenoxy)butyl]-3-tert.butyl-oxiran IIa/1. Nach $^1H$-NMR beträgt das Verhältnis von E zu Z-Verbindung 99:1.
$^1H$-NMR ($COCl_3$):
0.9 s 9H; 1.5-1.7 m 4H; 1.8-1.9 m 2H; 2.49 m 1H,
2.82 m 1H, 4.05 t 2H; 6.8-7.1 m 4H.

Tabelle 4a

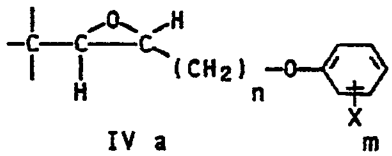

E-Konfiguration

IV a

| Nr. | $X_m$ | n | Physikalische Daten |
|---|---|---|---|
| IIa/1 | 2-F | 4 | $^1H$-NMR siehe Beispiel |
| IIa/2 | 2-Cl | 4 | |
| IIa/3 | 2-$CH_3$ | 4 | |
| IIa/4 | 4-F | 4 | |
| IIa/5 | 4-Cl | 4 | |
| IIa/6 | 4-$CH_3$ | 4 | |
| IIa/7 | 2,4-$Cl_2$ | 4 | |
| IIa/8 | 2,4,6-$Cl_3$ | 4 | |

Tabelle 4b

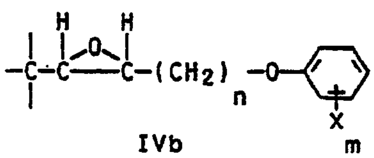

Z-Konfiguration

IVb

| Nr. | $X_m$ | n | Physikalische Daten |
|---|---|---|---|
| IIb/1 | 2-F | 4 | |
| IIb/2 | 2-Cl | 4 | |
| IIb/3 | 3-$CH_3$ | 4 | |
| IIb/4 | 4-F | 4 | |
| IIb/5 | 4-Cl | 4 | |
| IIb/6 | 4-$CH_3$ | 4 | |
| IIb/7 | 2,4-$Cl_2$ | 4 | |
| IIb/8 | 2,4,6-$Cl_3$ | 4 | |

Herstellung der Triazolverbindung

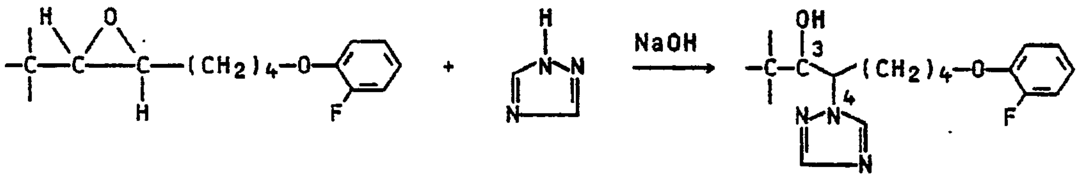


Ia/1 3R/4S-3S/4R-Enantiomerenpaar

9,5 g (0,036 mol) E-2-(2-Fluorphenoxybutyl)-3-t-butyloxiran IIa/1, ca. 90 %ig nach Gaschromatographie, 50 ml N-Methylpyrrolidon, 1,5 g (0,036 mol) Natriumhydroxid und 2,5 g (0,036 mol) 1,2,4-Triazol werden zusammengegeben und bei 180°C gerührt. Die Reaktion wird gaschromatographisch verfolgt. Nach 4 h ist der Umsatz größer als 98 %. Man destilliert N-Methylpyrrolidon am Hochvakuum ab und löst den Rückstand nach Erkalten in Wasser/Methylenchlorid. Die wäßrige Phase wird noch einmal mit Methylenchlorid extrahiert. Die vereinigten Methylenchloridphasen wäscht man mit Wasser säurefrei, trocknet sie über Natriumsulfat und engt ein. Das zurückbleibende braune Öl (10,9 g) wird in 150 ml Cyclohexan in der Wärme gelöst, 30 min mit 0,5 g Aktivkohle gerührt und heiß abfiltriert. Aus dem Filtrat erhält man nach Eindampfen 8,6 g 8-(2-Fluorphenoxy)-4-(1,2,4-triazol-1-yl)-3-hydroxy-2,2-dimethyloctan als farbloses Öl, welches beim Stehen kristallisiert. Das Diastereomerenverhältnis beträgt laut NMR-Spektrum (RR+SS):(RS+SR)=1:99. Smp.: 87°C.

<u>Tabelle 6a</u>

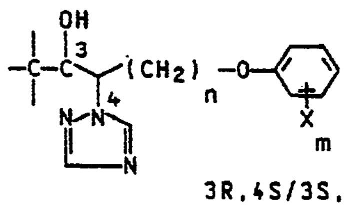

3R,4S/3S,4R Enantiomerenpaar

| Nr. | $X_m$ | n | Physikalische Daten |
|---|---|---|---|
| Ia/1 | 2-F | 4 | siehe Beispiel |
| Ia/2 | 2-Cl | 4 | |
| Ia/3 | 2-$CH_3$ | 4 | |
| Ia/4 | 4-F | 4 | |
| Ia/5 | 4-Cl | 4 | |
| Ia/6 | 4-$CH_3$ | 4 | |
| Ia/7 | 2,4-$Cl_2$ | 4 | |
| Ia/8 | 2,4,6-$Cl_3$ | 4 | |

Tabelle 6b

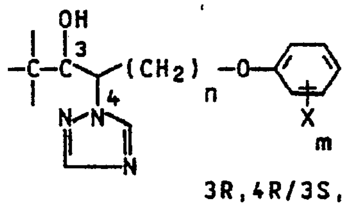

3R,4R/3S,4S Enantiomerenpaar

| Nr. | $X_m$ | n | Physikalische Daten |
|---|---|---|---|
| Ib/1 | 2-F | 4 | |
| Ib/2 | 2-Cl | 4 | |
| Ib/3 | 2-$CH_3$ | 4 | |
| Ib/4 | 4-F | 4 | |
| Ib/5 | 4-Cl | 4 | |
| Ib/6 | 4-$CH_3$ | 4 | |
| Ib/7 | 2,4-$Cl_2$ | 4 | |
| Ib/8 | 2,4,6-$Cl_3$ | 4 | |

## Patentansprüche

1. Verfahren zur Herstellung einer Verbindung der Formel I

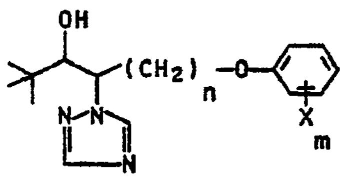

I

in der
X für Wasserstoff, Fluor, Chlor, Brom, $C_1$-$C_3$-Alkyl, $C_1$-$C_3$-Alkoxy, Trifluormethyl, Phenyl oder Phenoxy steht,
m eine ganze Zahl von 1 bis 3 ist, wobei die einzelnen Atome oder Gruppen gleich oder verschieden sind, wenn m größer als 1 ist, und
n eine ganze Zahl von 2 bis 6 bedeutet,
in Form ihrer weitgehend reinen Diastereomeren, dadurch gekennzeichnet, daß man ein Alkenoxid der Formel

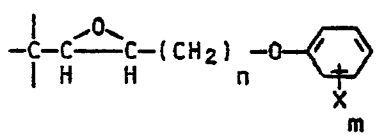

II

in der X, n und m die oben genannten Bedeutungen haben, in Form seines weitgehend reinen E- oder Z-Isomeren mit Triazol in Gegenwart eines Säureamids und einer alkalischen Verbindung bei einer Temperatur von 130 bis 230°C umsetzt.

2. Verfahren zur Herstellung eines Alkenoxids der Formel II

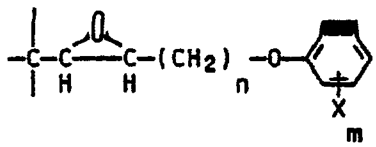

II

in der X, n und m die in Anspruch 1 genannten Bedeutungen haben, in Form seines weitgehend reinen E- oder Z-Isomeren, dadurch gekennzeichnet, daß man ein Alken der Formel III

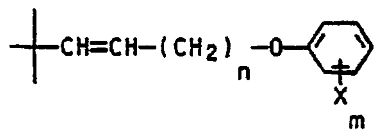

III

in der X, n und m die in Anspruch 1 genannten Bedeutungen haben, in Form seines weitgehend reinen E- oder Z-Isomeren mit einer organischen Persäure in Gegenwart einer organischen Flüssigkeit bei einer Temperatur von -10 bis +30°C oxydiert.

3. Verfahren zur Herstellung eines Alkens der Formel III

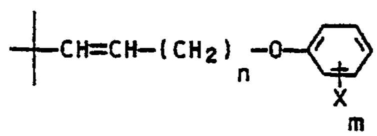

III

in der X, n und m die in Anspruch 1 genannten Bedeutungen haben, in Form seines weitgehend reinen E-Isomeren, dadurch gekennzeichnet, daß man eine Verbindung der Formel IV

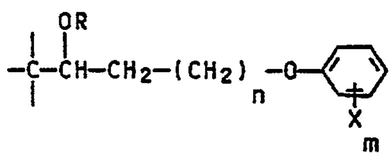

IV

in der X, n und m die in Anspruch 1 genannten Bedeutungen haben und R den Rest $-COCH_3$ oder $-CSSCH_3$ bedeutet, auf eine Temperatur von 200 bis 400°C oder 100 bis 300°C erhitzt.

4. Verfahren zur Herstellung eines Alkens der Formel III

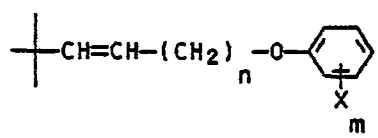

III

in der X, n und m die in Anspruch 1 genannten Bedeutungen haben, in Form seines weitgehend reinen Z-Isomeren, dadurch gekennzeichnet, daß man Pivalaldehyd der Formel $(CH_3)_3C-CHO$ mit einer Verbindung der Formel V

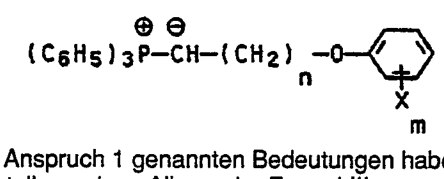

V

in der X, n und m die in Anspruch 1 genannten Bedeutungen haben umsetzt.

5. Verfahren zur Herstellung eines Alkens der Formel III

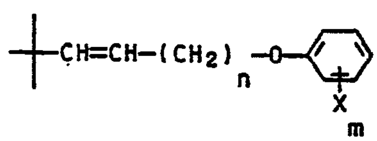

III

in der X, n und m die in Anspruch 1 genannten Bedeutungen haben, in Form seines weitgehend reinen E-Isomeren, dadurch gekennzeichnet, daß man ein Alken der Formel III

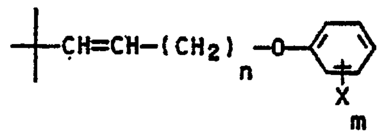

III

in der X, n und m die in Anspruch 1 genannten Bedeutungen haben, in Form seines Z-Isomeren mit kurzwelligem Licht in Gegenwart von Diphenyldisulfid und einem Kohlenwasserstoff behandelt.

6. Alkylenoxid der Formel II

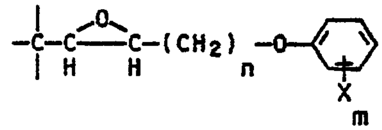

II

in der X, n und m die in Anspruch 1 genannten Bedeutungen haben.

7. Alken der Formel III

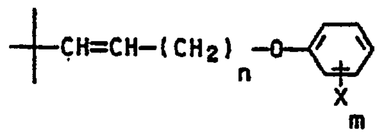

III

in der X, n und m die in Anspruch 1 genannten Bedeutungen haben.

8. Verbindung der Formel IV

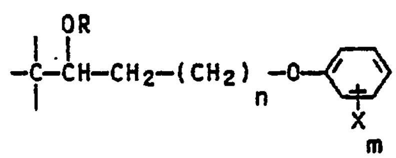

IV

in der X, n und m die in Anspruch 1 genannten Bedeutungen haben und R den Rest $-COCH_3$ oder $-CSSCH_3$ bedeutet.

**Claims**

1. A process for preparing a compound of the formula, I

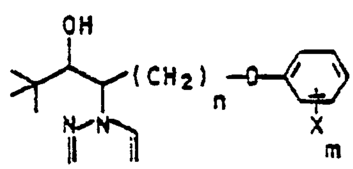

I

where
X is hydrogen, fluorine, chlorine, bromine, $C_1$–$C_3$-alkyl, $C_1$–$C_3$-alkoxy, trifluoromethyl, phenyl or phenoxy,
m is 1, 2 or 3, the individual atoms or groups being identical or different when m is greater than 1, and
n is 2, 3, 4, 5 or 6,
in the form of its substantially pure diastereomers, which comprises reacting an alkene oxide of the formula

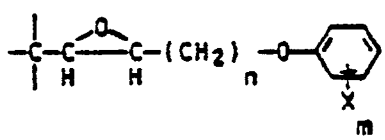

II

where X, n and m have the abovementioned meanings, in the form of its substantially pure E- or Z-isomer with triazole in the presence of an acid amide and of an alkaline compound at 130–230°C.

2. A process for preparing an alkene oxide of the formula II

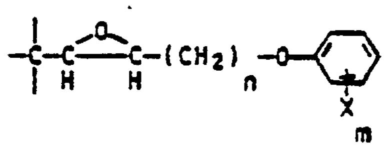

II

where X, n and m have the meanings mentioned in claim 1, in the form, of its substantially pure E- or Z-isomer, which comprises oxidizing an alkene of the formula III

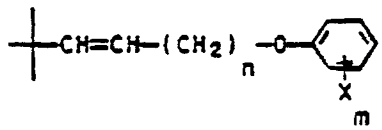

III

where X, n and m have the meanings mentioned in claim 1, in the form of its substantially pure E- or Z-isomer with an organic peracid in the presence of an organic liquid at from −10 to +30°C.

3. A process for preparing an alkene of the formula III

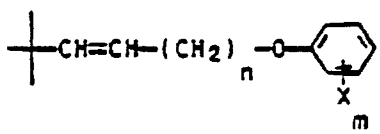

III

where X, n and m have the meanings mentioned in claim 1, in the form of its substantially pure E-isomer, which comprises heating a compound of the formula IV

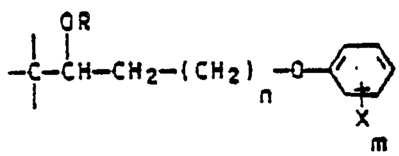

IV

where X, n and m have the meanings mentioned in claim 1 and R is $-COCH_3$ or $-CSSCH_3$, to 200–400°C or 100–300°C.

4. A process for preparing an alkene of the formula III

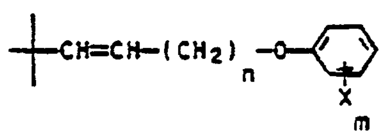

III

where X, n and m have the meanings mentioned in claim 1, in the form of its substantially pure Z-isomer, which comprises reacting pivalaldehyde of the formula $(CH_3)_3C-CHO$ with a compound of the formula V

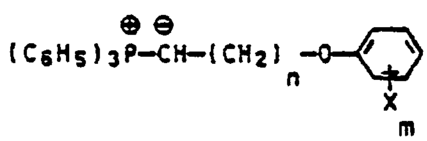

V

where X, n and m have the meanings mentioned in claim 1.

5. A process for preparing an alkene of the formula III

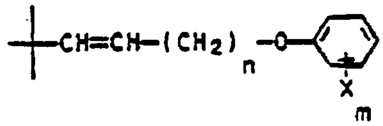

III

where X, n and m have the meanings mentioned in claim 1,
in the form of its substantially pure E-isomer, which comprises treating an alkene of the formula III

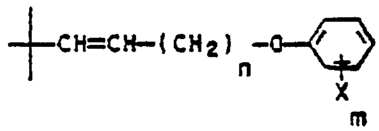

III

where X, n and m have the meanings mentioned in claim 1, in the form of its Z-isomer with short-wave light in the presence of diphenyl disulfide and of a hydrocarbon.

6. An alkylene oxide of the formula II

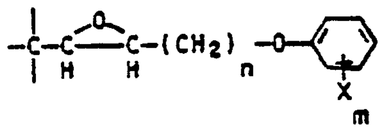

II

where X, n and m have the meanings mentioned in claim 1.

7. An alkene of the formula III

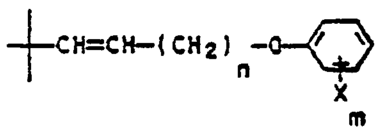

III

where X, n and m have the meanings mentioned in claim 1.

8. A compound of the formula IV

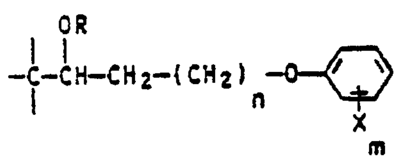

IV

where X, n and m have the meanings mentioned in claim 1 and R is $-COCH_3$ or $-CSSCH_3$.

**Revendications**

1. Procédé de préparation d'un composé de la formule I

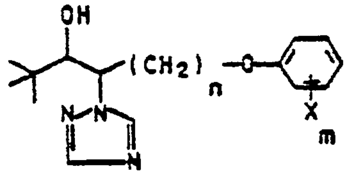

I

dans laquelle
X est mis pour hydrogène, fluor, chlore, brome, alkyle en $C_1$–$C_3$, alcoxy en $C_1$–$C_3$, trifluorométhyle, phényle ou phénoxy
m est un nombre entier de 1 à 3, les atomes ou groupes respectifs étant identiques ou différents, quand m est supérieur à 1,
n représente un nombre entier de 2 à 6
sous forme de ses diastereomères extrêmement purs, caractérisé par le fait qu'on fait réagir, à une température de 130 à 230 degrés C, un alcénoxyde de la formule

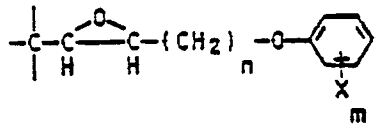 II

dans laquelle X, n et m ont les significations susindiquées, sous forme de son isomère E ou Z extrêmement pur, avec du triazole, en présence d'un amide d'acide et d'un composé alcalin.

2. Procédé de préparation d'un alcénoxyde de la formule II

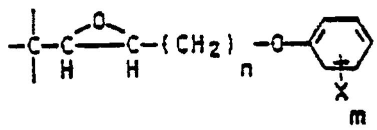 II

dans laquelle X, n et m ont les significations indiquées dans la revendication 1, sous forme de son isomère E ou Z extrêmement pur, caractérisé par le fait qu'on oxyde, à une température de -10 à + 30 degrés C un alcène de la formule III

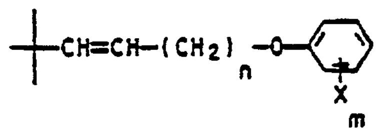 III

dans laquelle X, n et m ont les significations indiquées dans la revendication 1, sous forme de son isomère E ou Z, extrêmement pur, avec un péracide organique en présence d'un liquide organique.

3. Procédé de préparation d'un alcène de formule III

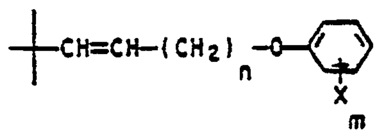 III

dans laquelle X, n et m ont les significations indiquées dans la revendication 1, sous forme de son isomère E, extrêmement pur, caractérisé par le fait qu'on chauffe à une température de 200 à 400 degrés C ou de 100 à 300 degrés C, un composé de formule IV

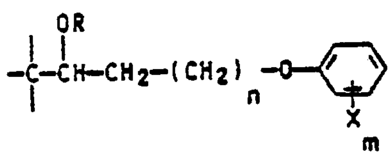 IV

dans laquelle X, n et m ont les significations données dans la revendication 1 et R représente le reste $-COCH_3$ ou $-CSSCH_3$.

4. Procédé de préparation d'un alcène de la formule III

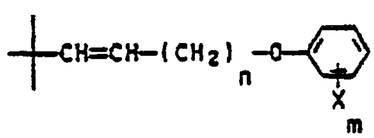 III

dans laquelle X, n et m ont les significations indiquées dans la revendication 1, sous forme de son isomère Z, extrêmement pur, caractérisé par le fait qu'on fait réagir de l'aldéhyde pivalique de la formule $(CH_3)_3C{-}CHO$ avec un composé de la formule V

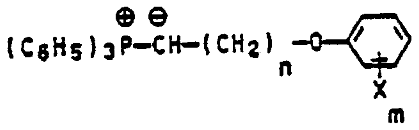

V

dans laquelle X, n et m ont les significations indiquées dans la revendication 1.

5. Procédé de préparation d'un alcène de formule III

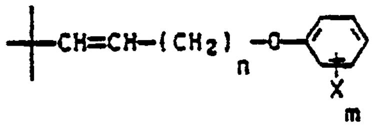

III

dans laquelle X, n et m ont les significations indiquées dans la revendication 1, sous forme de son isomère E, extrêmement pur, caractérisé par le fait qu'on traite un alcène de la formule III

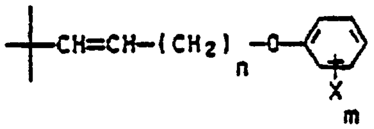

III

dans laquelle X, n et m ont les significations indiquées dans la revendication 1, sous forme de son isomère Z, extrêmement pur, avec une lumière d'ondes courtes en présence de bisulfure de diphényle et d'un hydrocarbure.

6. Alkylénoxydes de formule II

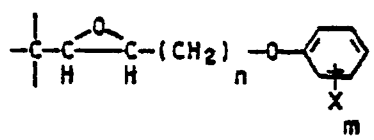

II

dans laquelle X, n et m ont les significations indiquées dans la revendication 1.

7. Alcène de la formule III

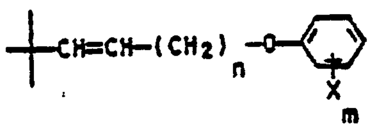

III

dans laquelle X, n et m ont les significations indiquées dans la revendication 1.

8. Composé de la formule IV

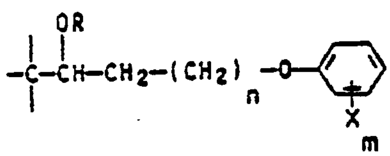

IV

dans laquelle X, n et m ont les significations données dans la revendication 1 et R représente le reste $-COCH_3$ ou $-CSSCH_3$.